# EUROPEAN PATENT APPLICATION

(11) **EP 4 806 479 A1**
(43) Date of publication of application: **16.09.2026**
(21) Application number: 24888782.0
(22) Date of filing: 07.11.2024
(51) Int. Cl.: A61M 5/303, A61M 5/30

(54) **NEEDLELESS SYRINGE**

(30) Priority: 07.11.2023 JP 2023190264
(71) Applicant: Daicel Corporation, Osaka-shi, Osaka 530-0011 (JP)
(72) Inventor: MITACHI, Takafumi, Tokyo 108-8230 (JP); MATSUMOTO, Yusuke, Tokyo 108-8230 (JP); IGA, Hiromitsu, Tokyo 108-8230 (JP)
(74) Representative: Grünecker Patent- und Rechtsanwälte PartG mbB
(86) International application number: PCT/JP2024/039627
(87) International publication number: WO 2025/100489

(57) **Abstract**

A needleless injector includes an injector assembly including a container configured to store an injectable substance, and a driver having a mechanism configured to pressurize the injectable substance to eject the injectable substance from a nozzle formed at a distal end of the container, a housing to which the injector assembly is attached, a sensor configured to detect a pressing force when the nozzle is pressed against an injection target region, and a control circuit configured to acquire the pressing force detected by the sensor and make a first decision that actuation of the driver is permitted when the pressing force reaches a first predetermined value or more.

## Description

### Technical Field

The present invention relates to a needleless injector.

### Background Art

In a needleless injector described in Patent Document 1, a protruding member protruding from an end surface of a housing is provided at a first position. When an ejection port is brought into contact with an injection target region by a user attempting injection and the protruding member is pressed and moved to a second position, this needleless injector applies a voltage to a drive unit to eject an injectable substance. Thus, the needleless injector in Patent Document 1 is configured to ignite an explosive in response to pressing operation by a user, thereby preventing ejection not intended by the user.

### Citation List

### Patent Document

Patent Document 1: JP 2015-150401 A

### Summary of Invention

### Technical Problem

When a needleless injector is used to administer an injection solution (injectable substance) to an injection target region under skin, the depth of administration may vary depending on the pressure with which a nozzle is pressed against the skin. For example, when the needleless injector is pressed with an appropriate pressing force equal to or greater than a predetermined value, the injectable substance is administered intradermally (to a shallow portion under the skin). In contrast, when the pressing force is excessively weak, the injectable substance may reach the muscle beyond the intradermal region. Here, in a case where a large amount of a substance that exhibits activity against an injection solution is present in the intradermal region, intradermal injection (to an injection target region) is desirable. However, if the pressure with which the needleless injector is pressed against the skin is excessively weak and thus the injection solution is administered so deeply as to reach the muscle, the effect of the injectable substance may not be sufficiently obtained.

The technique according to the present disclosure has been made in view of the above circumstances, and an object of the present disclosure is to provide a needleless injector that enables an injectable substance to be administered to a target depth.

### Solution to Problem

To solve the above problem, a needleless injector according to the present disclosure includes,
an injector assembly including a container configured to store an injectable substance, and a driver having a mechanism configured to pressurize the injectable substance to eject the injectable substance from a nozzle formed at a distal end of the container,
a housing to which the injector assembly is attached,
a sensor configured to detect a pressing force when the nozzle is pressed against an injection target region, and
a control circuit configured to acquire the pressing force detected by the sensor and make a first decision that actuation of the driver is permitted when the pressing force reaches a first predetermined value or greater.

The needleless injector may further include a notification unit configured to notify a user of a result of the first decision by the control circuit.

The control circuit may make a second decision that actuation of the driver is not permitted, when the pressing force detected by the sensor is equal to or greater than a second predetermined value greater than the first predetermined value.

The needleless injector may further include a notification unit configured to notify a user of a result of the first decision or a result of the second decision.

The notification unit may issue a first notification to the user when the pressing force is equal to or greater than the first predetermined value and less than the second predetermined value, and issues a second notification to the user when the pressing force is equal to or greater than the second predetermined value, and
contents of the first notification and the second notification may be different from each other.

The injector assembly may be detachably attached to the housing, and
the sensor may be disposed between the injector assembly and the housing.

In the needleless injector, a plurality of the sensors may be disposed at symmetrical positions about a center axis of the injector assembly.

In the needleless injector,
in the nozzle, a plurality of nozzle distal ends each including an ejection port of the injectable substance may be disposed about a predetermined axis along a pressing direction of pressing against the injection target region, and
the plurality of sensors may be disposed at symmetrical positions about the predetermined axis.

The control circuit may store, in a memory, log data indicating the pressing force when the injectable substance is ejected, display the log data on a display unit, or transmit the log data to an external device.

The control circuit may set the first predetermined value based on at least one of an input by a user, a type of the injectable substance, a type of the container, or an injection target.

The notification unit may be a display unit configured to display the pressing force in the form of a numerical value or a graph.

The control circuit may drive the driver to eject the injectable substance from the nozzle when the control circuit determines that actuation of the driver is permitted.

The control circuit may issue an advance notice of actuation to a user, after the control circuit makes the first decision and before an actuation signal is transmitted to the driver. Advantageous Effects of Invention

According to the present disclosure, it is possible to provide a needleless injector that enables an injectable substance to be administered to a target depth.

### Brief Description of Drawings

FIG. 1 is a diagram schematically illustrating the appearance of an injector.
FIG. 2 is a first cross-sectional view of the injector.
FIG. 3 is a second cross-sectional view of the injector.
FIG. 4 is a diagram illustrating a configuration of a housing 2 included in the injector.
FIG. 5 is a diagram illustrating a schematic configuration of an injector assembly.
FIG. 6 is a cross-sectional view illustrating a schematic configuration of a container.
FIG. 7 is an external view illustrating the schematic configuration of the container.
FIG. 8 is a diagram illustrating an arrangement of sensors provided on an accommodation space upper wall.
FIG. 9 is a diagram illustrating a configuration of a control circuit.
FIG. 10 is a diagram illustrating a control method executed by the control circuit of the injector.
FIG. 11 is a diagram illustrating a configuration of a nozzle according to a modified example.
FIG. 12 is a diagram illustrating arrangement of sensors in the modified example.
FIG. 13 is a diagram illustrating a control method according to a second embodiment.

### Description of Embodiments

### First Embodiment

With reference to the drawings, a needleless injector 1 according to an embodiment of the present disclosure (herein, simply referred to as "injector") is described below. The injector 1 is a needleless injector that ejects an ejection solution, which corresponds to an injectable substance of the present application, to an injection target region (hereinafter, simply referred to as target region), using combustion energy of an explosive. In other words, the injector 1 is a device that performs an injection by ejecting the ejection solution to the target region without using an injection needle.

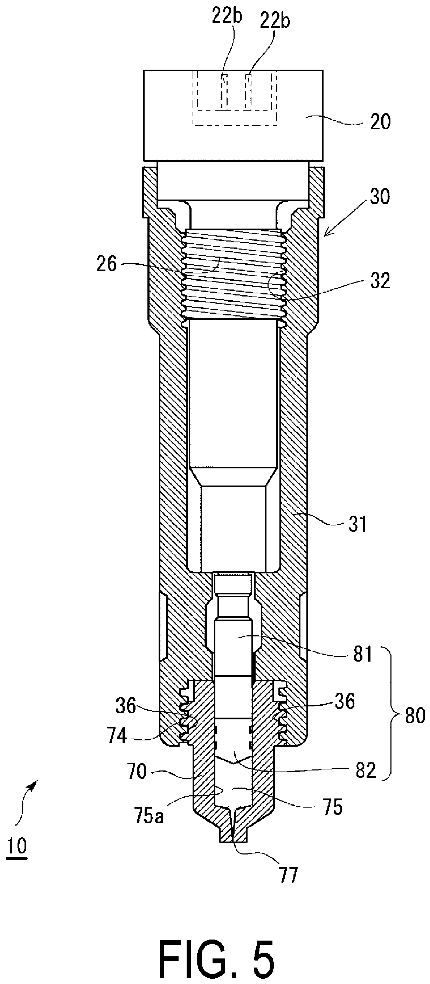

Note that each of the configurations, combinations thereof, and the like in each embodiment are examples, and various additions, omissions, substitutions, and other changes may be made as appropriate without departing from the spirit of the present disclosure. In addition, in the description of each embodiment, the injectable substance is referred to as an injection solution, but the form thereof is not intended to be limited, and for example, the injectable substance may be in a powder form. The present disclosure is not limited by the embodiments and is limited only by the claims. Note that, in the present embodiment, as terms indicating a relative positional relationship in a longitudinal direction of the injector 1, "distal end side" and "base end side" are used. The "distal end side" indicates a side closer to the distal end of the injector 1 described later, that is, a position closer to an ejection port 77, and the "base end side" indicates a side in an opposite direction to the "distal end side" in a longitudinal direction of the injector 1, that is, a direction to an igniter 22 side of an injector assembly 10 (see FIG. 5 described later).

### <Configuration of Injector>

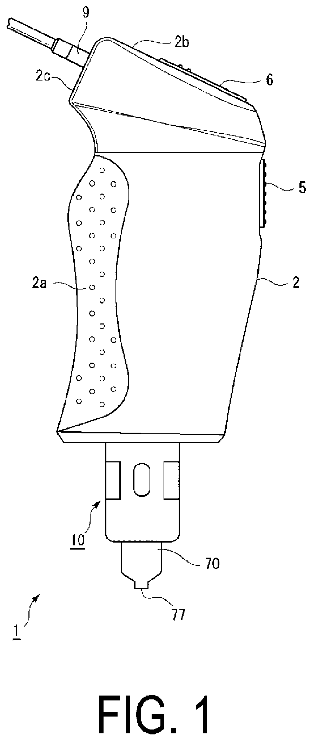

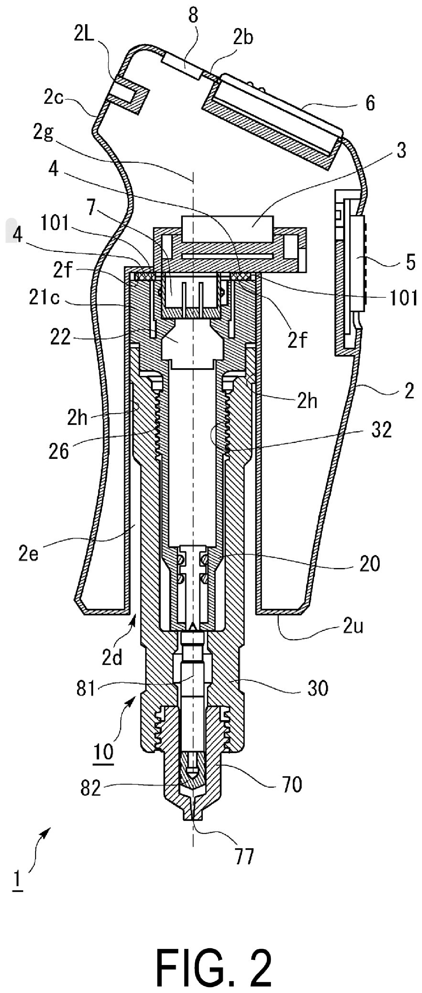

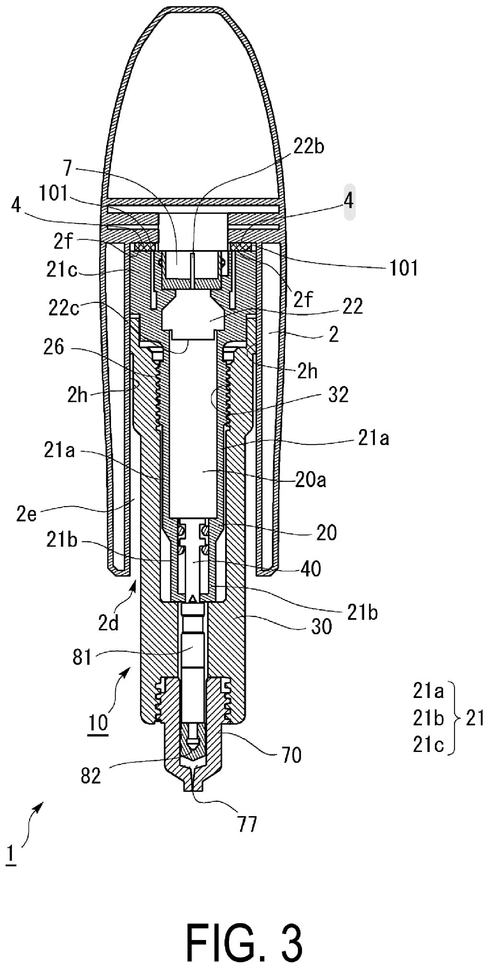

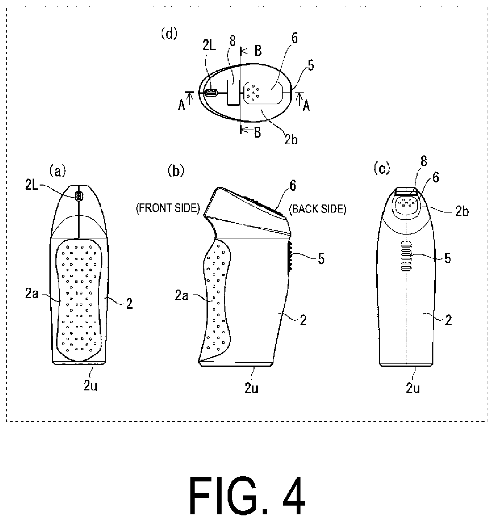

Here, FIG. 1 is a diagram schematically illustrating the appearance of the injector 1. FIG. 2 is a first cross-sectional view of the injector 1, which is an AA cross section in FIG. 4 described later. FIG. 3 is a second cross-sectional view of the injector 1, which is a BB cross section in FIG. 4 described later. The BB cross section is orthogonal to the AA cross section. FIG. 4 is a diagram illustrating a configuration of the housing 2 that is included in the injector 1, and FIG. 5 is a diagram illustrating a schematic configuration of the injector assembly 10. The injector 1 is formed by the injector assembly 10 being attached to the housing 2. The housing 2 contains a control circuit 3 and a sensor 4. A cable 9 (FIG. 1) for supplying a drive current to a driver 20 in the injector assembly 10 is connected to the housing 2.

The ejection solution ejected to the target region by the injector 1 is formed of a liquid medium including a predetermined substance, which exerts an effect or a function expected in the target region. In the ejection solution, the predetermined substance may be in a state of being dissolved in a liquid being a medium, or may be in a state of being simply mixed instead of being dissolved.

Examples of the predetermined substance included in the ejection solution include an organism-derived substance and a substance with a desired bioactivity, which can be ejected to the target region being an organism. Examples of the organism-derived substance include DNA, RNA, a nucleic acid, an antibody, and a cell. Examples of the substance with a bioactivity include various substances exerting pharmacological or therapeutic effects, which are exemplified by medicines composed of low-molecular-weight compounds, proteins, peptides, or the like, a vaccine, an inorganic substance such as metal particles for thermotherapy or radiotherapy, and a carrying body functioning as a carrier. Further, the liquid being the medium of the ejection solution may be any substance suitable for administering the predetermined substance exemplified by the aforementioned substances to the target region, regardless of whether it is aqueous or oleaginous. Further, the viscosity of the liquid being the medium is not particularly limited as long as the predetermined substance can be ejected by the injector 1.

In the injector 1, the injector assembly 10 is configured to be attachable to and detachable from the housing 2 freely. An accommodation space 75 (see FIG. 5) formed between a container (accommodation portion) 70 and a plunger 80 in the injector assembly 10 is filled with the ejection solution during a preparation stage, which is a stage before the actuation of the injector 1. The injector assembly 10 is a unit that is replaced each time the ejection solution is ejected. The injector assembly 10 will be described in detail below.

On the other hand, the housing 2 includes a grip portion 2a formed to be gripped by a user of the injector 1 in use, and is provided with a plurality of switches for operating the injector 1 to eject the ejection solution. Note that the injector 1 can be gripped and operated by one hand of the user. In this context, the housing 2 will be described with reference to FIG. 4. In FIG. 4, (a) illustrates the appearance of the housing 2 as viewed from the front side, (b) illustrates the appearance of the housing 2 as viewed from one side, (c) illustrates the appearance of the housing 2 as viewed from the back side, and (d) illustrates the appearance of the housing 2 as viewed from the uppers side. Here, the "front side" indicates a portion positioned on the distal side of the user holding the housing 2, which is the left side in FIG. 4(b), and the "back side" indicates a portion positioned on the proximal side of the user holding the housing 2, which is the right side in FIG. 4(b). Thus, when the user holds the housing 2 with one hand, the fingertips rest on the front side of the distal housing 2, which is the distal side, and the wrist is in the vicinity of the back side of the housing 2, which is the proximal side. The term "upper side" refers to a portion of the injector 1 on the base end side.

Considering the way in which the housing 2 is held by the user, the grip portion 2a is provided at a front side portion of the housing 2, and thus the user can easily grip the housing 2 with his or her fingertips. The grip portion 2a is provided with a plurality of dimples to improve the grip of the fingertips of the user. Furthermore, the grip portion 2a includes gentle recesses and protrusions on the front side of its outer shell (see FIG. 4(b)), facilitating gripping by the forefinger and middle finger of the user, for the sake of more stable holding of the housing by the user.

Further, the housing 2 is provided with a first switch 5 and a second switch 6 that are two operating switches for operating the injector 1. The first switch 5 and the second switch 6 are connected to a control unit, such as a microcontroller, as described below. The control unit controls the supply of ignition current to the igniter 22 based on a signal from each switch, thereby controlling an operation of the injector 1. The first switch 5 is a sliding switch provided on the back side of the housing 2, the sliding direction of which is an upward and downward direction of the housing 2 (direction connecting the distal end and the base end). The first switch 5 is constantly biased in the upward direction. The user can achieve a standby state of the injector 1 by continuously sliding the first switch 5 downward (toward the distal end side) for a predetermined period of time against the biasing force. Hereinafter, the operation of setting the injector 1 to the standby state is also referred to as a start operation. The standby state is a state in which the injector 1 is ready to eject the ejection solution, and a state in which the ejection is implemented when a user makes an additional operation in this state. When the injector 1 is in the standby state, the user can release the injector 1 from the standby state by continuously sliding the first switch 5 downward for a predetermined period of time against the biasing force.

The second switch 6 is a press type switch provided on an inclined surface 2b on the upper side of the housing 2. The user can press the second switch 6 in a direction toward the inner portion of the housing 2. The control unit is configured to supply an ignition current to the igniter 22 in response to the pressing operation on the second switch 6 while the injector 1 is in the standby state as a result of the start operation described above. A display unit 8 is provided at the inclined surface 2b on the upper side of the housing 2. The display unit 8 is a form of a notification unit that displays a decision result or the like by the control circuit 3 to notify the user. The notification unit is not limited to the display unit 8, and may be an indicator, a speaker, a communication module, or the like. Further, a connector 2L to which the cable 9 is connected is provided in a front inclined surface 2c on the upper side of the housing 2. In the present embodiment, the connector 2L is a USB connector, and the cable 9 is freely attachable to and detachable from the housing 2.

Note that, as described above, in the present embodiment, the power for actuating the igniter 22 is supplied from the outside through the cable 9. Alternatively, a battery for supplying such power may be provided on the inner portion of the housing 2. In this case, the housing 2 can be repeatedly used while replacing the injector assembly 10, until the battery runs out. When the battery runs out, the battery may be replaced or charged.

An opening 2d for inserting the injector assembly 10 is provided at a surface 2u (distal end surface) on the lower side of the housing 2, and an accommodation space 2e (FIG. 2) for the injector assembly 10 extends upward from the opening 2d. The accommodation space 2e has a shape corresponding to the injector assembly 10, and is substantially cylindrical in the present embodiment. A socket 7 and the sensor 4 are disposed at the upper end of the accommodation space 2e.

### <Injector Assembly>

The injector assembly 10 is attached to the housing 2 to form the injector 1, as illustrated in FIGS. 2 and 3. As illustrated in FIG. 5, the injector assembly 10 of the present embodiment is an assembly including the driver (drive unit) 20, an attachment 30, the container 70, and the plunger 80. How the injector assembly 10 is assembled will be described below.

The driver 20 includes a body 21 formed in a cylindrical shape. The body 21 includes a center portion 21a in the center thereof, a distal end portion 21b on the distal end side thereof, and a base end portion 21c on the base end side thereof. The distal end portion 21b, the center portion 21a, and the base end portion 21c of the body 21 have their internal spaces in communication with each other. The distal end portion 21b further has an opening on the distal end side. Here, the igniter 22, which is an electric igniter that generates ejection energy through combustion of an ignition agent, is attached to the base end portion 21c of the body 21. The igniter 22 includes an ignition pin 22b to which ignition current is supplied from the outside. The ignition pin 22b is coupled to a socket 7 on the side of the housing 2 in a state in which the injector assembly 10 is attached to the housing 2. The attachment state of the igniter 22 to the body 21 is determined such that a combustion product generated by the actuation of the igniter 22 is discharged toward the center portion 21a of the body 21. Specifically, the igniter 22 is attached to the base end portion 21c of the body 21 to have a discharge surface 22c, from which the combustion product is discharged, directed toward the center portion 21a.

Herein, a combustion energy used in the igniter 22 for the ignition agent is an energy for the injector 1 to eject the ejection solution to the target region. Note that, examples of the ignition agent include an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), an explosive containing aluminum and iron oxide (AFO), and an explosive composed of a combination of a plurality of these explosives. These explosives exhibit characteristics that, although the explosives generate high-temperature and high-pressure plasma during combustion immediately after ignition, when the combustion product condenses at room temperature, the explosives do not contain gaseous components and hence the pressure generated decreases abruptly. Note that an explosive other than these may be used as the ignition agent as long as appropriate ejection of the ejection solution can be performed.

The body 21 is a cylindrical member, and the internal space of the center portion 21a is a combustion chamber 20a. Furthermore, a male thread portion 26 is formed in a part of the outer surface of the center portion 21a. The male thread portion 26 is configured to be screwed into a female thread portion 32 of the attachment 30 described below. The effective lengths of the male thread portion 26 and the female thread portion 32 are determined in a manner that sufficient coupling force can be guaranteed therebetween. The internal space of the distal end portion 21b adjacent to the center portion 21a is formed in a cylindrical shape in which a piston 40 is slidably disposed.

Then, when the igniter 22 is actuated, the combustion product is discharged into the combustion chamber 20a, the pressure therein rises, and the piston 40 receives the pressure and results in sliding toward the distal end side. Thus, the driver 20 has a mechanism with the igniter 22 serving as an actuation source and the piston 40 serving as an output unit.

The attachment 30 is a member for attaching the driver 20, the plunger 80, and the container 70 as illustrated in FIG. 5. For a body 31 of the attachment 30, a resin such as nylon 6-12, polyarylate, polybutylene terephthalate, polyphenylene sulfide, or a liquid crystal polymer, which are known, may be used, for example. Further, filler such as glass fibers and glass filler may be contained in those resins. 20 to 80 mass% of glass fibers may be contained in polybutylene terephthalate, 20 to 80 mass% of glass fibers may be contained in polyphenylene sulfide, or 20 to 80 mass% of minerals may be contained in a liquid crystal polymer. Note that the material of the attachment 30 is not limited to resin, and may be metal or ceramic.

In the internal space of the body 31, in a region extending from the base end side to the center, the driver 20 is disposed as illustrated in FIG. 5. Then, in the internal space where the driver 20 is disposed, in a region on the base end side, the base end portion 21c of the driver 20 is generally positioned, and in a region on the distal end side whose diameter is smaller than that of the base end side, the center portion 21a and the distal end portion 21b of the driver 20 are generally positioned. Further, the female thread portion 32 is disposed on the inner wall surface of the body 31, and the female thread portion 32 is formed in a manner that the male thread portion 26 provided at the center portion 21a of the driver 20 can be screwed into the female thread portion 32.

Further, in the internal space of the body 31, in a region on the distal end side with respect to the region where the driver 20 is disposed, the plunger 80 is generally disposed as illustrated in FIG. 5. The diameter of the region where the plunger 80 is disposed is smaller than the diameter of the region where the distal end portion 21b of the driver 20 is disposed, and is a diameter that allows the plunger 80 to slide.

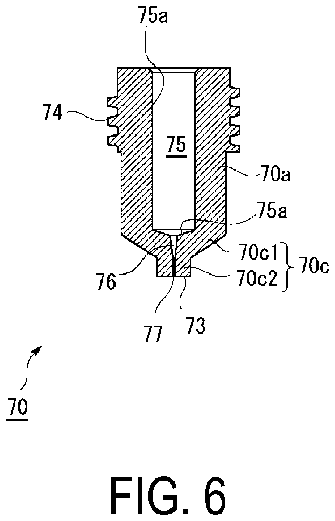

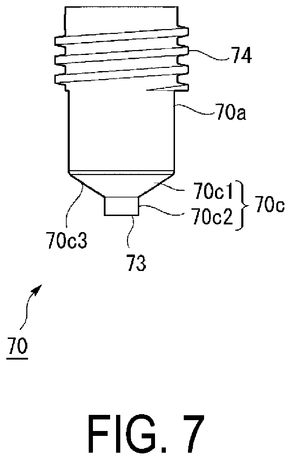

Further, in the internal space of the body 31, in a region on the distal end side, a part of the container 70 is generally disposed. The region where the container 70 is disposed communicates with the region where the plunger 80 is disposed on the base end side of the region, and the distal end side of the region where the container 70 is disposed is open to the distal end surface of the attachment 30. A female thread portion 36 for attaching the container 70 is formed at the inner wall of the region where the container 70 is disposed. The female thread portion 36 is screwed with a male thread portion 74 of the container 70 illustrated in FIGS. 6 and 7 described below, whereby the attachment 30 and the container 70 are coupled to each other.

Next, the plunger 80 will be described. The plunger 80 is a member that pressurizes the ejection solution using energy received from the piston 40, and includes a plunger rod 81 and a stopper portion 82. The plunger rod 81 is formed using, for example, a resin material suitable for pressurizing the plunger rod 81, but is not limited thereto, and may be formed using, for example, the same type of material as that of the attachment 30.

The stopper portion 82 formed using an elastic member such as rubber is attached to the distal end side of the plunger rod 81. Note that specific examples of the material of the stopper portion 82 include butyl rubber and silicone rubber. Further, there may be exemplified a styrene-based elastomer, a hydrogenated styrene-based elastomer, or a substance obtained by mixing a styrene-based elastomer or a hydrogenated styrene-based elastomer with polyolefin such as polyethylene, polypropylene, polybutene, or an α-olefin copolymer, oil such as liquid paraffin or process oil, or a powder inorganic substance such as talc, cast, or mica. Further, as the material of the stopper portion 82, a polyvinyl chloride-based elastomer, an olefin-based elastomer, a polyester-based elastomer, a polyamide-based elastomer, a polyurethane-based elastomer, any of various rubber materials (particularly, a vulcanized material) such as natural rubber, isoprene rubber, chloroprene rubber, nitrile butadiene rubber, and styrene butadiene rubber, or a mixture thereof may be employed. Furthermore, the stopper portion 82 pressurizes the ejection solution by sliding inside the container 70 described below. Thus, a surface of the stopper portion 82 and an inner wall surface 75a of the container 70 may be coated or processed using any of various substances, for the purposes of securing and adjusting slidability between the stopper portion 82 and the inner wall surface 75a of the accommodation space 75 of the container 70. Examples of the coating agent that can be used include polytetrafluoroethylene (PTFE), silicone oil, diamondlike carbon, and nanodiamond.

FIG. 6 is a cross-sectional view illustrating a schematic configuration of the container 70, and FIG. 7 is an external view illustrating the schematic configuration of the container 70. The container 70 is a member that accommodates the ejection solution and defines a flow path for ejecting, to the target region, the ejection solution pressurized by the plunger 80. The container 70 is formed using a material selected in consideration of the pressurization by the plunger and the definition of the flow path. The container 70 of the present embodiment is formed using a resin material. Note that the container 70 is not limited to being formed using the resin material, and may be formed using, for example, the same type of material as that of the attachment 30.

The container 70 includes the accommodation space 75 that can accommodate the ejection solution and is formed in a manner that the stopper portion 82 of the plunger 80 can move therein, and a flow path 76 that connects the accommodation space 75 to the ejection port (ejection portion) 77 facing the outside of the container 70. Specifically, the container 70 includes a body portion 70a having a cylindrical shape and defining the accommodation space 75, and a nozzle 70c that is connected to the distal end side of the body portion 70a and defines the flow path 76. Further, the nozzle 70c is configured to include a nozzle distal end portion 70c2 internally including a part of the flow path 76 on the ejection port 77 side, and a tapered portion 70c1 that connects the nozzle distal end portion 70c2 and the body portion 70a to each other and includes a distal end side outer surface 70c3 inclined with respect to the center axis in the longitudinal direction of the attachment 30. Then, a positional misalignment prevention tool 50 described below is externally fitted to the outer periphery of the nozzle distal end portion 70c2.

In the injector assembly 10, as illustrated in FIG. 5, the distal end side of the plunger 80 is fitted into the accommodation space 75 of the container 70 in a manner that the stopper portion 82 of the plunger 80 can slide inside the accommodation space 75 in a direction toward the nozzle 70c (direction toward the distal end side). In a state where the plunger 80 is fitted into the container 70, a space formed between the stopper portion 82 of the plunger 80 and the container 70 becomes a storage space of the ejection solution, that is, a space in which the ejection solution is encapsulated. In other words, when the plunger 80 is fitted into the initial position of the accommodation space 75 in the container 70, the distal end surface of the stopper portion 82 and the inner wall surface, of the container 70, located closer to the distal end side than the distal end surface of the stopper portion 82 define the storage space. The flow path of the container 70 opens in a distal end surface 73 of the nozzle 70c to form the ejection port 77. Therefore, when the plunger 80 slides inside the accommodation space 75, the ejection solution accommodated in the accommodation space 75 is pressurized and the ejection solution is ejected from the ejection port 77 through the flow path 76.

The flow path 76 provided in the container 70 has an inner diameter smaller than that of the accommodation space 75. With this configuration, the ejection solution that has been applied with a high pressure is ejected to the outside through the ejection port 77. Further, the male thread portion 74 for attaching the container 70 to the attachment 30 is formed on the outer periphery on the base end side of the container 70. The male thread portion 74 can be screwed into the female thread portion 36 of the attachment 30.

The distal end shape of the stopper portion 82 of the plunger 80 is formed generally matching the distal end shape of the accommodation space 75, which is defined by the inner wall surface 75a near a portion at which the accommodation space 75 and the flow path 76 are connected to each other (the deepest part of the accommodation space 75). In the present embodiment, both the distal end shape of the stopper portion 82 and the distal end shape of the accommodation space 75 have a tapered shape that decreases in diameter toward the distal end side. With this configuration, a smallest possible gap can be formed between the stopper portion 82 and the inner wall surface 75a of the container 70 when the plunger 80 slides for ejecting the ejection solution and reaches the deepest part of the accommodation space 75, whereby the ejection solution can be prevented from wastefully remaining in the accommodation space 75. However, the shape of the stopper portion 82 is not limited to a particular shape as long as desired effects can be obtained with the injector 1 of the present embodiment. Further, the stopper portion 82 is formed having an outer diameter slightly larger than that of the accommodation space 75 of the container 70, and thus, when the stopper portion 82 is fitted into the accommodation space 75 in a state of being compressed in the radial direction, the stopper portion 82 suitably comes into contact with the inner wall surface 75a in a manner that the airtightness is maintained between the stopper portion 82 and the container 70. Note that the stopper portion 82 need not necessarily be formed to have a larger outer diameter than the accommodation space 75 and, for example, may have substantially the same diameter as the accommodation space 75, as long as the ejection solution can be appropriately ejected.

### <Assembly of Injector>

With regard to the assembly of the injector 1, first, how the injector assembly 10 is assembled will be described. In a state where the stopper portion 82 of the plunger 80 is inserted to the deepest part of the accommodation space 75 of the container 70, the plunger 80 is retracted with the ejection port 77 of the container 70 in communication with the ejection solution. Since the stopper portion 82 and the inner wall surface 75a of the accommodation space 75 are in close contact with each other, the retraction action produces a negative pressure in the accommodation space. Thus, the accommodation space 75 can be filled with the ejection solution through the ejection port 77. At this time, the plunger 80 is retracted to such an extent as to make the part of the plunger 80 protruding from the container 70 (plunger rod 81) reach the region where the piston 40 is disposed in the internal space of the attachment 30, when the container 70 is attached to the attachment 30 in this state.

When the container 70 with the accommodation space 75 filled with the ejection solution is attached to the attachment 30, the driver 20 is further inserted into the attachment 30 from the base end side. The driver 20 is inserted until the distal end surface of the piston 40 disposed at the distal end portion 21b of the driver 20 reaches the base end surface of the plunger 80 inside the attachment 30. At this time, the male thread portion 26 provided at the center portion 21a of the driver 20 is screwed into the female thread portion 32 of the attachment 30, whereby the driver 20 and the attachment 30 are suitably coupled to each other. At this time, the piston 40 incorporated in the driver 20 is connected to the plunger 80. Note that the connection between the piston 40 and the plunger 80 is not limited to simple abutment, and fitting portions may be provided at the distal end of the piston 40 and the base end of the plunger 80 and fitted to each other.

When the driver 20 is attached to the attachment 30 to which the container 70 and the plunger 80 are attached as described above, the plunger 80 is pushed in a manner that it moves from the piston 40 toward the distal end side, whereby the plunger 80 is positioned at a predetermined position (pre-actuation position) inside the container 70. At this time, in accordance with the pushing of the plunger 80, the excess ejection solution in the container 70 is discharged from the ejection port 77, and a predetermined amount of the ejection solution remains in the container 70. Note that the volume of the space for accommodating the ejection solution that is formed between the plunger 80 positioned at the pre-actuation position and the container 70 is determined and set to a volume that secures an appropriate ejection amount of the ejection solution when the injector 1 is actuated. Therefore, when the injector assembly 10 is assembled as described above, an amount of the ejection solution accommodated in the accommodation space 75 of the container 70, that is, an amount of the ejection solution to be ejected is set to the predetermined amount determined in advance.

The injector assembly 10 having the above-described configuration is attached to the housing 2, with a portion of the injector assembly 10 on the base end side inserted into the accommodation space 2e of the housing 2 and with the ignition pin 22b of the igniter 22 fitted into the socket 7 on the housing 2 side. At this time, a base end surface 101 of the injector assembly 10 abuts against the sensor 4. The injector assembly 10 may be configured to be held and prevented from falling off the housing 2 when the ignition pin 22b is fitted into the socket 7 on the housing 2 side. The injector assembly 10 may be configured to be held and prevented from falling off the housing 2, by the outer wall of the injector assembly 10 and the inner wall 2h defining the accommodation space 2e coming into contact and being interference-fitted together.

In this manner, the injector assembly 10 is loaded into the housing 2, whereby the injector 1 is prepared to be usable (see FIGS. 1 to 3). The user holds the housing 2 of the injector 1 with one hand and slides the first switch 5 located on the back side of the housing 2 for a predetermined period of time, putting the injector 1 in the standby state. Then, the user presses the nozzle 70c against the target region, and presses the second switch 6 when a notification that the pressing force is appropriate is issued, as described later. As a result, the igniter 22 is actuated, the ejection solution is pressurized via the piston 40 and the plunger 80, and the ejection solution is ejected from the ejection port 77 and injected into the target region.

### <Sensor>

The sensor 4 detects a pressing force when the nozzle 70c of the injector 1 is pressed against the injection target region, and inputs the pressing force to the control circuit 3. The sensor 4 includes, for example, a piezoelectric element which converts the magnitude of deformation when pressure is applied, into an electrical signal. Alternatively, the sensor 4 may be a sensor that detects a change in capacitance due to deformation when pressure is applied to a cell having a plurality of electrodes. Note that the method for detecting pressure by the sensor 4 is not particularly limited, and any method that can input, to the control circuit 3, an electric signal indicating a pressure value may be used. When the injector 1 is brought into a standby state as a result of the start operation performed on the first switch 5, the sensor 4 repeatedly detects the pressure and inputs the detection result to the control circuit 3, at intervals of about a few milliseconds to one second, for example.

The sensor 4 is provided at an accommodation space upper wall 2f located on the upper side of the accommodation space 2e of the housing 2. The sensor 4 is located between the base end of the injector assembly 10 and the accommodation space upper wall 2f when the injector assembly 10 is accommodated in the accommodation space 2e. That is, the sensor 4 is disposed between the container 70 attached to the injector assembly 10 and the accommodation space upper wall 2f of the housing 2. When the user presses, against the injection target region, the nozzle 70c at the distal end of the injector 1, a reactive force is transmitted from the base end of the injector assembly 10 to the sensor 4, and the sensor 4 detects the reactive force (pressing force).

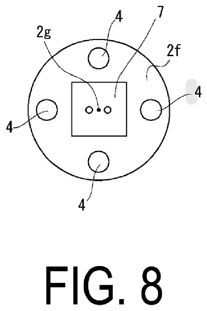

FIG. 8 is a diagram illustrating an arrangement example of the sensor 4 provided on the accommodation space upper wall 2f. As illustrated in FIG. 8, a plurality of (four in this example) sensors 4 are provided and disposed at positions rotationally symmetric about a predetermined axis 2g. The predetermined axis 2g of the present embodiment coincides with the center axis of the accommodation space 2e. Further, since the accommodation space 2e has a shape similar to the shape of the base end side portion of the injector assembly 10, the predetermined axis 2g also coincides with the center axis of the injector assembly 10 and the center axis of the container 70. That is, the sensors 4 are disposed at symmetrical positions about the center axis of the container 70. In this configuration, when the injector 1 is pressed perpendicularly against the surface of the target region, the plurality of sensors 4 output substantially the same detection values, whereas when the injector 1 is pressed diagonally against the surface of the target region, the plurality of sensors 4 output different detection values. Therefore, based on the detection value of each sensor 4, the control circuit can determine whether the injector 1 is pressed correctly, i.e., perpendicularly against the surface of the target region.

### <Control Circuit>

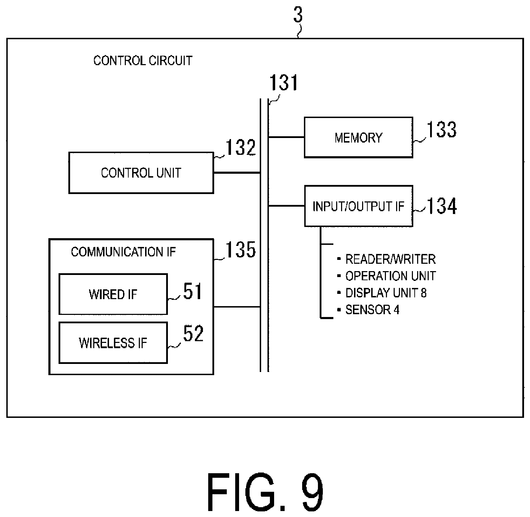

FIG. 9 is a diagram illustrating the configuration of the control circuit 3. The control circuit 3 is a computer including a control unit 132, a memory (storage device) 133, an input/output interface (IF) 134, and a communication IF 135, which are connected to each other by a connection bus 131. The control unit 132 processes input information and outputs a processing result, thereby performing control of the entire device, and the like. The control unit 132 is also referred to as a central processing unit (CPU) or a micro-processing unit (MPU). The control unit 132 is not limited to a single processor, and may have a multiprocessor configuration. Further, the control unit 132 may have a multi-core configuration in which a plurality of cores are provided in a single chip to be connected using a single socket.

The memory 133 may be a main storage device or an auxiliary storage device. The main storage device is used as, for example, a work area for the control unit 132, a storage area for temporarily storing information to be processed by the control unit 132, and a buffer area for communication data. That is, the main storage device is a storage medium used by the control unit 132 to cache programs and data and/or as a work area. The main storage device includes, for example, a random access memory (RAM), a read only memory (ROM), and a flash memory. The auxiliary storage device is a storage medium that stores programs to be executed by the control unit 132, data used for information processing, operation setting information, and the like. The auxiliary storage device is, for example, a solid state drive (SSD), an erasable programmable ROM (EPROM), a flash memory, a USB memory, a memory card, or the like. The auxiliary storage device may store setting information of the injector 1, a determination condition, a history (log) of the detected pressing force, and the like.

The input/output IF 134 is an interface for inputting/outputting data between the control circuit 3 and peripheral devices connected to the control circuit 3. The input/output IF 134 performs data exchange between the control circuit 3 and devices such as a reader/writer that reads and writes data from and to a storage medium such as a flash memory or an SSD, an operation unit, a display unit, a speaker, and a sensor. The operation unit is an input unit to which information for the control circuit 3 is input through a user operation, such as an operation button, a selection key, or a touch screen. The display unit 8 is an output unit that outputs, to the user, information such as a determination result from the control unit 132 by displaying the information. The touch screen may be disposed over a display region of a display device, and may be configured to detect a touch operation on an icon or the like displayed on the display device and input the detection result to the control circuit 3. The operation unit of the present embodiment includes the first switch 5 and the second switch 6. The operation unit may be operated by the user to input a first predetermined value and a second predetermined value to the control circuit 3 to set these values. Alternatively, statistical data and/or a data table may be stored in the memory in advance, and when the user operates the operation unit to input information on the injectable substance (type and amount of the liquid medicine to be used, the type of container, and the like) and/or information on the administration target (sex, age, body fat percentage, location of administration, and the like), the control circuit 3 may set the first predetermined value and/or the second predetermined value based on the input information.

The communication IF 135 is an interface (communication module) that communicates with another device via a communication link (network), and is also referred to as a communication control unit (CCU). The communication IF 135 of the present embodiment includes a wired IF 51 for performing wire communication and a wireless IF 52 for performing wireless communication. The wired IF 51 communicates with other devices via the cable 9, for example. The wireless IF 52 communicates with other devices via, for example, a WLAN. Although the wireless IF 52 of the present embodiment employs a communication link defined by IEEE 802.11, the wireless communication is not limited thereto, and a communication link according to Bluetooth (trade name) may be used, for example.

In the control circuit 3, the control unit 132 realizes functions described later based on an application program. That is, the control unit 132 realizes required functions by software. However, some or all of the functions may be realized by hardware such as a dedicated large scale integration (LSI) such as a digital signal processor (DSP), an application specific integrated circuit (ASIC), or a field-programmable gate array (FPGA); a logic circuit; or other digital circuits. At least a part of the hardware may include an analog circuit. The control unit 132 may be configured to include one processor that functions as a plurality of process units, or may be configured to include a plurality of processors each functioning as one process unit.

The control circuit 3 acquires the pressing force detected by the sensor 4, and when the pressing force reaches the first predetermined value or greater, the control circuit 3 makes a first decision that actuation of the driver 20 is permitted. The control circuit 3 makes a second decision that actuation of the driver 20 is not permitted, when the pressing force detected by the sensor 4 exceeds the second predetermined value greater than the first predetermined value. The control circuit 3 notifies the user of the results of the first decision and the second decision by displaying the results on the display unit 8. Note that the notification to the user is not limited to the display by the display unit 8, and may be a notification by sound output from a speaker, information transmission from the CCU to the user terminal, vibration by a vibrator, or the like.

The control circuit 3 issues a first notification to the user when the pressing force is equal to or greater than the first predetermined value and does not exceed the second predetermined value. Then, the control circuit 3 issues a second notification to the user, when the pressing force is equal to or greater than the second predetermined value or when the pressing force is equal to or smaller than the first predetermined value. The control circuit 3 causes the display unit 8 to display characters, for example, "actuation permitted" as the first notification or "actuation not permitted" as the second notification. The control circuit 3 may output, from the speaker, "actuation permitted" and "actuation not permitted" in the form of an audible message. The control circuit 3 may also use colors on an indicator to indicate corresponding notifications, such as "green" as the first notification and "red" as the second notification. In this manner, the control circuit 3 issues the first notification and the second notification which have different contents, and can thereby accurately notify the user that the pressing force has reached to a level at which injection is permitted (first notification) and that an excessive or insufficient pressing force is being applied and thus the ejection is not permitted (second notification).

### <Control Method>

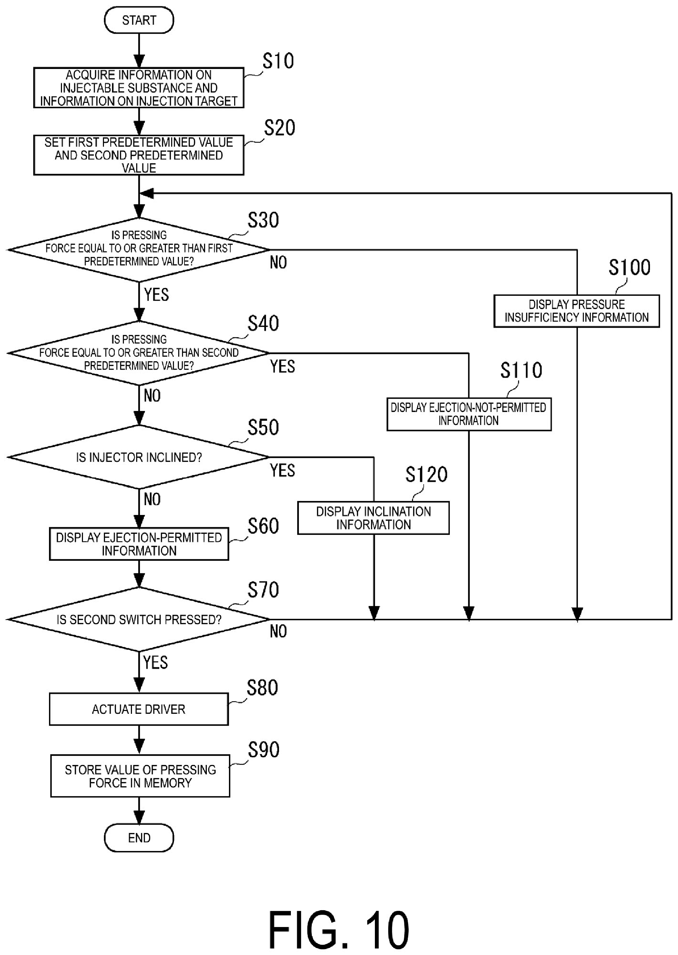

FIG. 10 is a diagram illustrating a control method executed by the control circuit 3 of the injector 1. When the user performs the start operation and the injector 1 is brought into in the standby state, the control circuit 3 starts the processing depicted in FIG. 10.

In step S10, the control circuit 3 acquires information on the injectable substance and information on the injection target. For example, the control circuit 3 acquires information on the injectable substance and information on the injection target which are input from the operation unit by the user. The control circuit 3 may acquire the information on the injectable substance and the information on the injection target from another device, via the cable 9 or a wireless communication link.

In step S20, the control circuit 3 sets the first predetermined value and second predetermined value, based on the information on the injectable substance and the information on the injection target acquired in step S10. Note that the second predetermined value is greater than the first predetermined value and is, for example, 60 N. The first and second predetermined values are not limited thereto, and the first predetermined value may be from 3 N to 10 N, and the second predetermined value may be from 30 N to 80 N.

In step S30, the control circuit 3 acquires the pressing force detected by the sensor 4, and determines whether actuation of the driver 20 is permitted based on whether the pressing force has reached the first predetermined value or greater. If the pressing force is equal to or greater than the first predetermined value and the determination is positive, the control circuit 3 proceeds to step S40, and if the determination is negative, the control circuit 3 proceeds to step S100.

In step S40, the control circuit 3 acquires the pressing force detected by the sensor 4, and determines whether actuation of the driver 20 is permitted, based on whether the pressing force is equal to or greater than the second predetermined value or whether the pressing force is less than the first predetermined value after decision that the pressing force is equal to or greater than the first predetermined value is made. If it is determined that the pressing force is less than the second predetermined value, the control circuit 3 proceeds to step S50. If it is determined that the pressing force is equal to or greater than the second predetermined value, the control circuit 3 proceeds to step S110.

In step S50, the control circuit 3 determines whether the injector 1 is being pressed in a state of being inclined, based on the detection values of the plurality of sensors 4. If the differences between the detection values of the sensors 4 are less than a predetermined value, the control circuit 3 determines that the injector 1 is not inclined and is appropriately pressed, and the control circuit 3 proceeds to step S60. On the other hand, if the differences between the detection values of the sensors 4 are equal to or greater than the predetermined value, the control circuit 3 determines that the injector 1 is pressed in a state of being inclined, and the control circuit 3 proceeds to S120.

In step S60, the control circuit 3 causes the display unit 8 to display information indicating that ejection is permitted (ejection-permitted information) as the first notification. Examples of the ejection-permitted information include characters such as "ejection permitted", a numerical value of the pressing force, and a graph indicating the pressing force. The first notification may be, for example, changing the color of characters or background being displayed to a specific color (for example, green), outputting a specific sound, or a combination thereof.

In step S70, the control circuit 3 determines whether the second switch 6 is pressed, and if the determination is positive, the control circuit 3 proceeds to step S80, and if the determination is negative, the control circuit 3 proceeds to step S30.

In step S80, the control circuit 3 supplies a drive current (ignition current) to the driver 20 to actuate the driver 20.

In step S90, the control circuit 3 acquires the pressing force detected by the sensors 4 at the time of ejection, and stores the detection value of the pressing force, in the memory 133 as a log. The configuration of the control circuit 3 is not limited thereto, and the control circuit 3 may be configured to display the log data on the display unit 8 or transmit the log data to an external device. Such a configuration of the control circuit 3 makes it possible to check, at a later time, whether the ejection was performed with an appropriate pressing force.

If the determination is negative in step S30 and the process proceeds to step S100, the control circuit 3 causes the display unit 8 to display pressure insufficiency information as a notification indicating that ejection is not permitted due to insufficiency in the pressing force. Examples of the pressure insufficiency information include characters such as "insufficient pressing force", a value of the pressing force, and a graph indicating the pressing force. The notification of insufficient pressing force is not limited to the above display, and may be, for example, changing the color of characters or background being displayed to a specific color (for example, yellow), outputting an error sound, or a combination thereof.

If the determination is positive in step S40 and the process proceeds to step S110, based on the fact that the pressing force is out of the predetermined range, the control circuit 3 causes the display unit 8 to display ejection-not-permitted information as the second notification. Examples of the ejection-not-permitted information include characters such as "ejection not permitted", a numerical value of the pressing force, and a graph indicating the pressing force. The second notification may be, for example, changing the color of characters or background being displayed to a specific color (for example, red), outputting an error sound, or a combination thereof.

If the determination is positive in step S50 and the process proceeds to step S120, the control circuit 3 causes the display unit 8 to display inclination information indicating that the injector 1 is pressed in a state of being inclined and the injector 1 is inclined. Examples of the inclination information include characters such as "excessively inclined" and an icon indicating that the injector 1 is inclined. The notification of inclination may be, for example, changing the color of characters or background being displayed to a specific color (for example, blue), outputting an error sound, or a combination thereof. Further, guidance indicating the direction in which the injector is to be moved to correct the orientation of the injector can be displayed on the display unit 8.

### <Effects of Embodiment>

(1) The injector 1 of the present embodiment makes the first decision that actuation of the driver 20 is permitted, when the pressing force applied to the injection target region reaches the first predetermined value or greater. Thus, the injector 1 of the present embodiment can eject the injectable substance in a state of being pressed against the injection target region with an appropriate pressing force, and can administer the injectable substance to a target depth.
(2) The injector 1 further includes a notification unit that notifies a user of a result of the first decision by the control circuit 3. Thus, the injector 1 can enable the user to recognize an appropriate pressing force.
(3) The control circuit 3 of the injector 1 makes the second decision that actuation of the driver 20 is not permitted, when the pressing force detected by the sensor 4 is equal to or greater than the second predetermined value greater than the first predetermined value. Thus, the injector 1 can perform injection with an appropriate pressing force, and enables prevention of damage to the container or the like, reduction of a load applied to the injector 1, and avoidance of a failure.
(4) The injector 1 further includes a notification unit that notifies a user of a result of the first decision or a result of the second decision. Thus, the injector 1 enables the user to recognize that the pressing force is excessive, and can avoid damage or failure due to an excessive pressing force.
(5) The notification unit of the injector 1 issues the first notification when the pressing force is equal to or greater than the first predetermined value and less than the second predetermined value, and issues the second notification when the pressing force is equal to or greater than the second predetermined value. The contents of the first notification and the second notification are different from each other. Thus, the injector 1 can enable the user to recognize the range of the appropriate pressing force.
(6) The injector assembly 10 is detachably attached to the housing 2, and the sensor 4 is disposed between the injector assembly 10 and the housing 2. Thus, the sensor 4 is prevented from directly being in contact with the injection target region, and hygienic injection can be achieved.
(7) In the injector 1, a plurality of the sensors 4 are disposed at symmetrical positions about a center axis of the injector assembly 10. Thus, the sensors 4 can detect inclination of the injector 1 with respect to the injection target region. The injector 1 notifies the user of this inclination to allow the user to recognize the inclination. Thus, the user can perform injection with the injector 1 being in a correct orientation that is perpendicular to the surface of the injection target region.
(8) The control circuit 3 stores, in a memory, log data indicating the pressing force when the injectable substance is ejected, displays the log data on the display unit 8, or transmits the log data to an external device. Thus, the injector 1 can keep a record of the pressing force at the time of injection, and enables the user to check, at a later time, whether the injection was performed appropriately.
(9) The control circuit 3 can set the first predetermined value based on at least one of an input by a user, a type of the injectable substance, a type of the container, or an injection target. Thus, in the injector 1, each predetermined value can be precisely set through user operation on an operation unit.
(10) The notification unit of the injector 1 is a display unit that displays the pressing force in the form of a numerical value or a graph. Thus, the injector 1 can provide a numerical value or a graph that enables the user to accurately realize the pressing force.

### <Modified Examples>

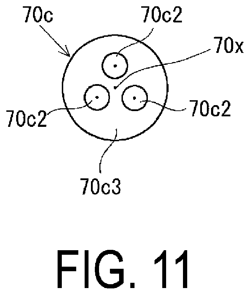

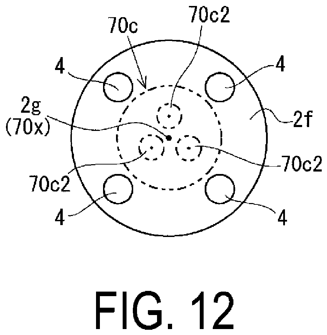

FIG. 11 is a diagram illustrating a configuration of the nozzle 70c according to a modified example, and FIG. 12 is a diagram illustrating a positional relationship between the sensors 4 and the nozzle distal end portions 70c2 in the modified example. FIG. 12 illustrates the accommodation space upper wall 2f and the nozzle distal end portion 70c2 in a superimposed manner, as if the accommodation space upper wall 2f to which the sensors 4 are attached is seen from the distal end side of the injector assembly 10 loaded into the accommodation space 2e. Note that, in FIG. 12, other components such as the socket 7, the body 21, and the attachment 30 are omitted. In FIG. 12, the nozzle distal end portions 70c2 are indicated by two dot-dash lines. The present modified example is different from the first embodiment described above in that a plurality of the nozzle distal end portions 70c2 are provided. Note that since other portions are the same as those of the first embodiment described above, the same elements are denoted by the same reference signs and description thereof will not be repeated.

As illustrated in FIG. 11, in the present modified example, the plurality of nozzle distal end portions 70c2 are provided at the distal end of the nozzle 70c. Although FIG. 11 illustrates an example in which three nozzle distal end portions 70c2 are provided, the number of the nozzle distal end portions 70c2 is not limited thereto, and may be two or four or more. The plurality of nozzle distal end portions 70c2 are disposed about a predetermined axis 70x along the pressing direction of the injector 1.

As illustrated in FIG. 12, the predetermined axis 70x coincides with the predetermined axis 2g located at the center of the plurality of sensors 4. That is, the plurality of sensors 4 are disposed at positions that are rotationally symmetric about the predetermined axis 70x. Thus, the injector of the present modified example can detect inclination of the injector 1 with respect to the injection target region. By notifying a user of this inclination, the injector 1 enables the user to perform injection with the injector 1 being in a correct orientation that is perpendicular to the surface of the injection target region.

### <Second Embodiment>

The present embodiment is different from the first embodiment described above in that the control circuit 3 performs ejection at the timing when a state where ejection is permitted is reached, instead of performing ejection in response to pressing of the second switch 6. Note that since other portions are the same as those of the first embodiment described above, the same elements are denoted by the same reference signs and description thereof will not be repeated.

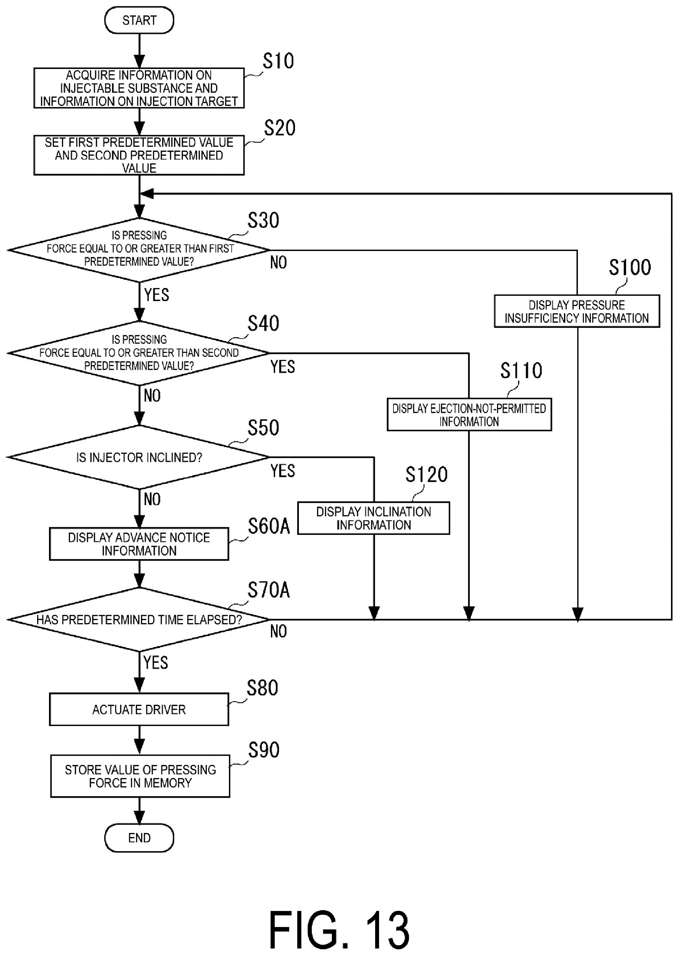

FIG. 13 is a diagram illustrating a control method according to a second embodiment. In FIG. 13, the processes from step S10 to step S50 are the same as the processes in FIG. 10.

If it is determined that the injector is not inclined in step S50 and the process proceeds to step S60A, the control circuit 3 outputs information as an advance notice of ejection (advance notice information). For example, the display unit 8 is caused to perform display output. Examples of the output of the advance notice information include display of characters such as "start ejection timer after five seconds" and "ejection preparation completed", and countdown to ejection. The output of the advance notice information may be, for example, changing the color of characters or background being displayed to a specific color (for example, purple), outputting a specific sound or voice, or a combination thereof.

In step S70A, the control circuit 3 determines whether a predetermined time has elapsed since the first advance notice was issued, and if the determination is positive, the control circuit 3 proceeds to step S80, and if the determination is negative, the control circuit 3 proceeds to step S30. Note that the processes from step S80 to step S120 are the same as the processes in FIG. 10.

As described above, when the control circuit 3 of the present embodiment determines that actuation of the driver 20 is permitted, the control circuit 3 drives the driver 20 to eject the injectable substance from the nozzle 70c. As a result, the injector 1 of the present embodiment automatically operates when the conditions for ejection are satisfied, and thus makes it possible to perform reproducible injection.

The control circuit 3 of the present embodiment issues an advance notice of actuation to a user, after the control circuit 3 makes the first decision and before an actuation signal is transmitted to the driver 20. Thus, the injector 1 of the present embodiment can inform the user of the timing of the actuation, and enables the user to prepare himself/herself mentally, and thus, injection can be performed without failure.

### Reference Signs List

1: Injector
2: Housing
3: Control circuit
4: Sensor
5: First switch
6: Second switch
7: Socket
8: Display unit
9: Cable
10: Injector assembly
20: Driver
20a: Combustion chamber
21: Body
21a: Center portion
21b: Distal end portion
21c: Base end portion
22: Igniter
22b: Ignition pin
22c: Discharge surface
26: Male thread portion
30: Attachment
31: Body
32: Female thread portion
36: Female thread portion
40: Piston
50: Positional misalignment prevention tool
70: Container
70a: Body portion
70c: Nozzle
70c1: Tapered portion
70c2: Nozzle distal end portion
70c3: Distal end side outer surface
70x: Predetermined axis
73: Distal end surface
74: Male thread portion
75: Accommodation space
75a: Inner wall surface
76: Flow path
77: Ejection port
80: Plunger
81: Plunger rod
82: Stopper portion
101: Base end surface
131: Connection bus
132: Control unit
133: Memory
134: Input/output IF
135: Communication IF
51: Wired IF
52: Wireless IF

## Claims

1. A needleless injector comprising:
an injector assembly including a container configured to store an injectable substance, and a driver having a mechanism configured to pressurize the injectable substance to eject the injectable substance from a nozzle formed at a distal end of the container;
a housing to which the injector assembly is attached;
a sensor configured to detect a pressing force when the nozzle is pressed against an injection target region; and
a control circuit configured to acquire the pressing force detected by the sensor and make a first decision that actuation of the driver is permitted when the pressing force reaches a first predetermined value or greater.

2. The needleless injector according to claim 1, further comprising:
a notification unit configured to notify a user of a result of the first decision by the control circuit.

3. The needleless injector according to claim 1, wherein the control circuit makes a second decision that actuation of the driver is not permitted, when the pressing force detected by the sensor is equal to or greater than a second predetermined value greater than the first predetermined value.

4. The needleless injector according to claim 3, further comprising:
a notification unit configured to notify a user of a result of the first decision or a result of the second decision.

5. The needleless injector according to claim 4, wherein
the notification unit issues a first notification to the user when the pressing force is equal to or greater than the first predetermined value and less than the second predetermined value, and issues a second notification to the user when the pressing force is equal to or greater than the second predetermined value, and
contents of the first notification and the second notification are different from each other.

6. The needleless injector according to claim 1, wherein
the injector assembly is detachably attached to the housing, and
the sensor is disposed between the injector assembly and the housing.

7. The needleless injector according to claim 6, wherein a plurality of the sensors are disposed at symmetrical positions about a center axis of the injector assembly.

8. The needleless injector according to claim 6, wherein
in the nozzle, a plurality of nozzle distal ends each including an ejection port of the injectable substance are disposed about a predetermined axis along a pressing direction of pressing against the injection target region, and
the plurality of sensors are disposed at symmetrical positions about the predetermined axis.

9. The needleless injector according to claim 1, wherein the control circuit stores, in a memory, log data indicating the pressing force when the injectable substance is ejected, displays the log data on a display unit, or transmits the log data to an external device.

10. The needleless injector according to claim 1, wherein the control circuit is configured to set the first predetermined value based on at least one of an input by a user, a type of the injectable substance, a type of the container, or an injection target.

11. The needleless injector according to claim 2, 4, or 5, wherein the notification unit is a display unit configured to display the pressing force in the form of a numerical value or a graph.

12. The needleless injector according to any one of claims 1 to 10, wherein the control circuit drives the driver to eject the injectable substance from the nozzle when the control circuit determines that actuation of the driver is permitted.

13. The needleless injector according to claim 12, wherein the control circuit issues an advance notice of actuation to a user, after the control circuit makes the first decision and before an actuation signal is transmitted to the driver.
